# EUROPEAN PATENT SPECIFICATION

(11) **EP 4 217 016 B1**
(45) Date of publication and mention of the grant of the patent: **11.11.2026**
(21) Application number: 21727579.1
(22) Date of filing: 18.05.2021
(51) Int. Cl.: A61M 1/06

(54) **PUMPING GARMENT**
PUMPKLEIDUNGSSTÜCK
VÊTEMENT POUR TIRAGE DU LAIT

(30) Priority: 23.09.2020 US 202063082092 P
(43) Date of publication of application: 02.08.2023
(60) Divisional application: 23169197.3 / 4 223 167
(73) Proprietor: Medela AG, 6340 Baar (CH)
(72) Inventor: STANTON, Lisa, Toronto, Ontario M9B 0B1 (CA)
(74) Representative: Grünecker Patent- und Rechtsanwälte PartG mbB
(86) International application number: PCT/IB2021/054274
(87) International publication number: WO 2022/064283

(56) References cited:
- US-A1- 2010 159 801
- US-A1- 2016 007 659
- US-A1- 2016 021 939

## Description

### CROSS-REFERENCE TO RELATED APPLICATION

This application claims the benefit of the filing date of U.S. Provisional Patent Application No. 63/082092, filed September 23, 2020, entitled "Pumping Garment."

### FIELD OF THE DISCLOSURE

This disclosure relates generally to a garment for a nursing woman using a breast pump to extract milk from her breasts for storage and subsequent feeding of an infant. The garment supports the portion of a breast pump system known as a breast shield, freeing the nursing woman's hands to carry out other tasks besides holding one or two breast shields in place while pumping. Such a pumping garment is known from US 2016/007659 A1, US 2010/159801 A1, and US 2010/021939 A1.

### BACKGROUND

Breastfeeding of an infant provides benefits to the infant and the breastfeeding woman. Breastmilk contains nutrients a baby needs in the amounts required for optimal growth and development and may provide additional health benefits, such as fewer digestive issues and fewer rashes. For the breastfeeding woman, breastfeeding reduces post-delivery bleeding and chances of anemia, aids in the uterus returning to its original size after birth, and burns up to an extra 500 calories per day.

However, not every nursing woman is able to breastfeed an infant on demand due to work schedules and other time demands. Accordingly, many nursing women use a breast pump to extract and store milk for feeding an infant. Breast pump systems routinely include a breast shield, which is a funnel-like apparatus having a conical region that is placed against the breast with the nipple in the center of the breast shield. Upon the application of negative pressure, the nipple is drawn toward, and often into, a tubular portion of the breast shield known as the nipple tunnel. The nipple tunnel of the breast shield is connected to other components of a breastmilk collection kit. This connection permits application of intermittent (i.e., cyclical) negative pressure to the interior of the breast shield, and also provides a flow path for breastmilk expressed into the nipple tunnel to be collected in a collection receptacle. The collection receptacle may be a breastmilk container having a threaded cap that can serve as a bottle for feeding an infant. A nursing woman generally has to manually hold the breast shield against her breast in order to pump milk.

Predictably, holding the breast shield in place is inconvenient and limits the tasks that the woman could otherwise accomplish while pumping. Although various brassieres and bustiers have been developed to hold the breast shield in place, such brassieres have drawbacks. For example, existing pumping garments often have apertures designed such that the breast shields must be inserted into the apertures from an interior of the garment, which is inconvenient and cumbersome for the wearer of the garment.

### SUMMARY

The invention is set out in the appended set of claims. According to an aspect of the disclosure, a pumping garment includes a top band and a main body below the top band. The main body includes a primary piece, a panel overlapping the primary piece, and a securement edge. The primary piece has a lateral boundary. The lateral boundary has a securement portion extending along the securement edge of the main body. The lateral boundary further has an aperture portion extending away from the securement edge of the main body. The panel includes a securement side extending along the securement edge of the main body. The panel further includes an aperture side spaced away from the securement edge of the main body. The aperture portion of the lateral boundary of the primary piece and the aperture side of the panel together define an aperture for supporting a breast shield.

According to another aspect of the disclosure, a pumping garment includes a top band and a main body below the top band. The main body includes a primary piece and a panel overlapping the primary piece. The primary piece includes an upper section and a bottom band. The upper section consists of a single ply material. The bottom band is seamlessly connected to the upper section and comprises two ply of material. The panel includes a bottom side sewn adjacent to the bottom band of the primary piece.

In some arrangements, the main body may include a first panel overlapping the primary piece, a second panel overlapping the primary piece, a first securement edge, and a second securement edge. The primary piece may include a first lateral boundary having a first securement portion extending along the first securement edge of the main body and a first aperture portion extending away from the first securement edge of the main body. The primary piece may further include a second lateral boundary having a second securement portion extending along the second securement edge of the main body and a second aperture portion extending away from the second securement edge of the main body. The first panel may include a first securement side extending along the first securement edge of the main body and a first aperture side spaced away from the first securement edge of the main body. The second panel may include a second securement side extending along the second securement edge of the main body and a second aperture side spaced away from the second securement edge of the main body. The first aperture portion of the first lateral boundary of the primary piece and the first aperture side of the first panel may together define a first aperture for supporting a breast shield. The second aperture portion of the second lateral boundary of the primary piece and the second aperture side of the second panel may together define a second aperture for supporting a breast shield.

In some arrangements, the aperture portion of the lateral boundary of the primary piece may curve away from the securement edge of the main body to form a symmetric arc. The securement portion of the lateral boundary of the primary piece may extend along the securement edge of the main body for a first distance beginning at a top of the securement edge and for a second distance beginning at a bottom of the securement edge. The aperture portion of the lateral boundary of the primary piece may be located between the first distance and the second distance.

In some arrangements, the aperture side of the panel may curve toward the securement side of the panel. A maximum distance between the aperture portion of the lateral boundary of the primary piece and the securement edge of the main body may be less than a minimum width of the panel. The aperture portion of the lateral boundary of the primary piece may be connected to the aperture side of the panel by a bar tack.

In some arrangements, a first zipper side and a zipper facing may be connected to the first securement edge, and a second zipper side may be connected to the second securement edge. A first fastener may be connected to the first securement edge, and a second fastener may be connected to the second securement edge.

In some arrangements, the main body may include a first panel overlapping the primary piece, a first securement edge, a second panel overlapping the primary piece, and a second securement edge. The first panel may include a first bottom side sewn adjacent to the bottom band of the primary piece and a first securement side extending along the first securement edge of the main body. The second panel may include a second bottom side sewn adjacent to the bottom band of the primary piece and a second securement side extending along the second securement edge of the main body. The bottom band may comprise a ribbed material.

### BRIEF DESCRIPTION OF THE DRAWINGS

While the specification concludes with claims particularly pointing out and distinctly claiming the subject matter that is regarded as the present disclosure, it is believed that the disclosure will be more fully understood from the following description taken in conjunction with the accompanying drawings. Some of the figures may have been simplified by the omission of selected elements for the purpose of more clearly showing other elements. Such omissions of elements in some figures are not necessarily indicative of the presence or absence of particular elements in any of the exemplary embodiments, except as may be explicitly delineated in the corresponding written description. None of the drawings are necessarily to scale.
Figure 1 illustrates a front perspective view of a pumping garment of the present disclosure in a fastened state.
Figure 2 illustrates a rear perspective view of the pumping garment depicted in Figure 1 in a fastened state.
Figure 3 illustrates a primary piece and two panels forming a main body of the pumping garment depicted in Figures 1 and 2.
Figure 4 illustrates a perspective view of the pumping garment depicted in Figures 1-3 on a wearer.
Figure 5 illustrates a perspective view of the pumping garment depicted in Figures 1-4 on a wearer and supporting a breast shield.
Figure 6 illustrates an exterior view of the pumping garment depicted in Figures 1-5 in an unfastened state.
Figure 7 illustrates an interior view of the pumping garment depicted in Figures 1-6 in an unfastened state.

### DETAILED DESCRIPTION OF THE DRAWINGS

The pumping garment disclosed herein provides numerous benefits. The apertures to support breast shields are formed by panels overlapping a primary piece, and this arrangement allows relatively simple manufacturing of the pumping garment. The primary piece and/or panels have curved edges or sides at the aperture, and the shape of these component pieces of pumping garment reduces material waste, prevents the garment from being unnecessarily bulky when worn, and allows breast shields to easily be inserted into the pumping garment from an exterior of the pumping garment. In particular, the curved edges forming the apertures are beneficial in that less material must be displaced in order to insert a breast shield, and therefore less material is bunched in a way that might interfere with pumping after the breast shields are inserted, than would be the case if panels with only straight edges were used. Further, the bottom band is seamlessly connected to the pumping garment, which further simplifies construction of the garment and eliminates uncomfortable chafing when the pumping garment is worn caused by seams located under the bust.

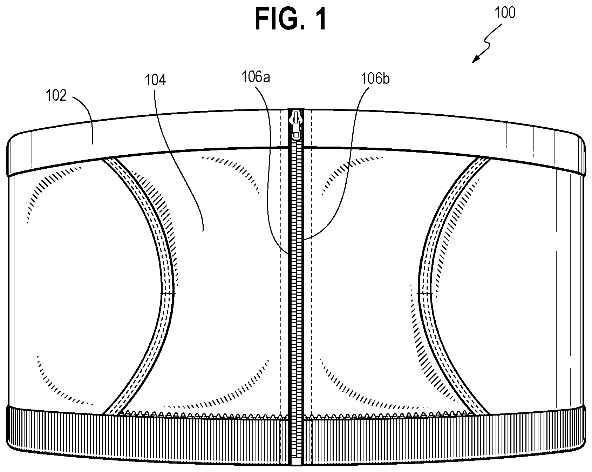

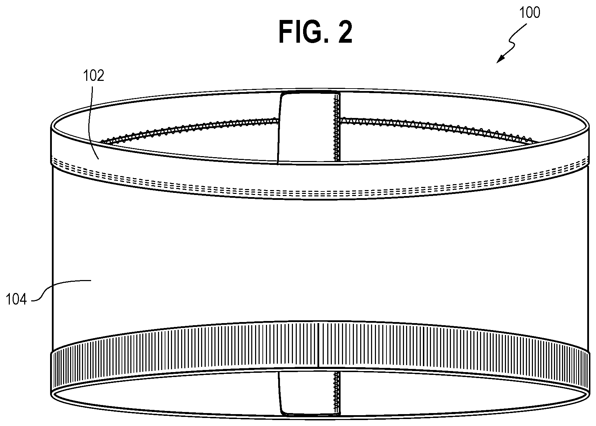

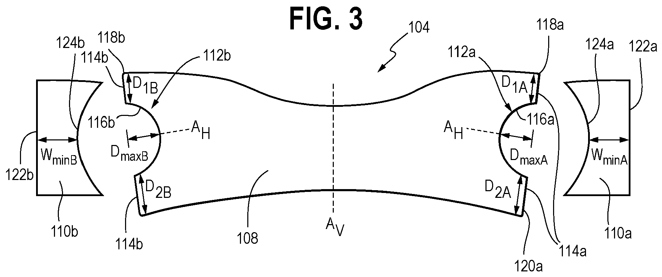

As shown in FIGS. 1 and 2, the pumping garment 100 is a strapless bandeau style brassiere. Although shown without straps, in other arrangements not herein depicted, bra straps may be provided that are permanently or removably connected to the pumping garment 100 and configured to be worn over the shoulders of a wearer to further secure the pumping garment 100 in place on a wearer. The pumping garment 100 includes a top band 102, a main body 104 below the top band 102, a first securement edge 106a, and a second securement edge 106b. The first securement edge 106a and the second securement edge 106b are lateral boundaries of the main body 104 that are selectively connectable to one another. As discussed below with respect to FIG. 3, the first securement edge 106a and the second securement edge 106b may be formed by multiple pieces of fabric. When the pumping garment 100 is worn by a wearer, the first securement edge 106a is connected, directly or indirectly, to the second securement edge 106b. For example, the first securement edge 106a may be zipped to the second securement edge 106b, as shown. Other options for fastening the first securement edge 106a to the second securement edge 106b, such as by snapping, buttoning, hooking clasps, or other standard means of fastening a garment, are also possible.

FIG. 3 shows the component pieces of material forming the main body 104. As shown in FIG. 3, the main body 104 includes a primary piece 108, a first panel 110a, and a second panel 110b. FIG. 3 shows the primary piece 108, the first panel 110a, and the second panel 110b prior to being connected to form the main body 104. The primary piece 108, the first panel 110a, and the second panel 110b are shown as unitary, integral pieces of fabric. However, in alternate arrangements not herein depicted, each of the primary piece 108, the first panel 110a, and the second panel 110b could comprise multiple pieces of material connected to include their respective characteristics and shapes (described further below). For example, the primary piece 108 could have a portion, particularly the portion that will cover the back of the wearer during use, that is formed from a different material than the portions of the primary piece 108 that will cover the front of the wearer during use. This could be advantageous because the wearer may prefer to have a cool, lightweight material cover their back and a thicker, more supportive material cover their front. The primary piece 108, the first panel 110a, and the second panel 110b may each comprise the same material or may be formed from different materials.

The primary piece 108 includes a first lateral boundary 112a and a second lateral boundary 112b. The first lateral boundary 112a includes a first securement portion 114a that, when the main body 104 is assembled, extends along the first securement edge 106a of the main body 104 (shown in FIG. 1). Returning to FIG. 3, the first lateral boundary 112a also includes a first aperture portion 116a that extends away from the first securement edge 106a of the main body 104 when the main body 104 is assembled (shown in FIG. 1). Similarly, as shown in FIG. 3, the second lateral boundary 112b includes a second securement portion 114b that, when the main body 104 is assembled, extends along the second securement edge 106b of the main body 104 (shown in FIG. 1). Returning to FIG. 3, the second lateral boundary 112b also includes a second aperture portion 116b that extends away from the second securement edge 106b of the main body 104 when the main body 104 is assembled (shown in FIG. 1).

In particular, as shown in FIG. 3, the first aperture portion 116a of the first lateral boundary 112a and the second aperture portion 116b of the second lateral boundary 112b curve away from, respectively, the first securement edge 106a and the second securement edge 106b (shown in FIG. 1) in respective symmetric arcs, each arc having a generally horizontal line of symmetry A_{H} through the main body 104. In first aperture portion 116a and the second aperture portion 116b are also symmetric relative to one another across a vertical line A_{V} of symmetry through the main body 104. In other arrangements not depicted herein, the first aperture portion 116a and the second aperture portion 116b may not be symmetric across a generally horizontal line of symmetry A_{H} through the main body 104 and may also not be symmetric relative to one another across a vertical line of symmetry A_{V} through the main body 104. As indicated in broken lines in FIG. 3, the generally horizontal lines of symmetry A_{H} and vertical line of symmetry A_{V} are virtual lines of symmetry, as opposed to actual visible seams or lines of stitching of the garment. Alternatively, the first aperture portion 116a and the second aperture portion 116b may have eccentric curvatures or may extend away from the first securement edge 106a and the second securement edge 106b (shown in FIG. 1), respectively, via a series of straight segments (e.g., straight segments forming half of a rectangle, pentagon, hexagon, or the like).

As shown in FIG. 3, the first securement portion 114a of the first lateral boundary 112a extends along the first securement edge 106a of the main body 104 (shown in FIG. 1) for a first distance D_{1A} beginning at a top 118a of the first securement edge 106a. As also shown in FIG. 3, the first securement portion 114a of the first lateral boundary 112a extends along the first securement edge 106a of the main body 104 (shown in FIG. 1) for a second distance D_{2A} beginning at a bottom 120a of the first securement edge 106a. The first aperture portion 116a of the first lateral boundary 112a is located between the first distance D_{1A} and the second distance D_{2A}. Also shown in FIG. 3, the second securement portion 114b of the second lateral boundary 112b extends along the second securement edge 106b (shown in FIG. 1) of the main body 104 for a first distance D_{1B} beginning at a top 118a of the second securement edge 106b. As also shown in FIG. 3, the second securement portion 114b of the second lateral boundary 112b also extends along the second securement edge 106b of the main body 104 (shown in FIG. 1) for a second distance D_{2B} beginning at a bottom 120b of the second securement edge 106b. The second aperture portion 116b of the second lateral boundary 112b is located between the first distance D_{1B} and the second distance D_{2B}.

In other arrangements not shown, the first securement portion 114a and the second securement portion 114b may extend along the first securement edge 106a and the second securement edge 106b, respectively, for only a single distance. The single distance may begin at the tops 118a or 118b, the bottoms 120a or120b, or in between the top 118a and the bottom 120a or the top 118b and the bottom 120b.

As illustrated in FIG. 3, first panel 110a has a first securement side 122a and a first aperture side 124a. Similarly, the second panel 110b has a second securement side 122b and a second aperture side 124b. The first securement side 122a extends along the first securement edge 106a of the main body 4 (illustrated in FIG. 1). Similarly, the second securement side 122b extends along the second securement edge 106b of the main body (illustrated in FIG. 1). The first securement side 122a and the second securement side 122b are shown as straight sides that extend continuously along the first securement edge 106a and the second securement edge 106b, respectively, of the main body 104. The continuous arrangement allows for simple manufacture and assembly of the first panel 110a and the second panel 110b. However, the first securement side 122a and the second securement side 122b may extend discontinuously along the first securement edge 106a and the second securement edge 106b. In particular, the first securement side 122a may extend along the first securement edge 106a only in areas where the first securement portion 114a of the first lateral boundary 112a of the primary piece 108 does not extend. Likewise, the second securement side 122b may extend along the second securement edge 106b only in areas where the second securement portion 114b of the second lateral boundary 112a of the primary piece does not extend. The discontinuous arrangement allows for material savings as less material is needed to form the first panel 110a and the second panel 110b.

As illustrated in FIG. 3, the first aperture side 124a of the first panel 110a is spaced away from the first securement edge 106a of the main body 4 (shown in FIG. 1), and the second aperture side 124b of the second panel 110b is spaced away from the second securement edge 106a of the main body 4 (illustrated in FIG. 1). Returning to FIG. 3, the first aperture side 124a curves toward the first securement side 122a, and the second aperture side 124b curves toward the second securement side 122b. The first aperture side 124a and the second aperture side 124b may curve along their entire lengths, as shown in FIG. 3, or may only curve along a portion of their lengths.

As illustrated in FIG. 3, the first panel 110a has a minimum width W_{minA} between the first securement side122a and the first aperture side 124a. Likewise, the second panel 110b has a minimum width W_{minB} between the second securement side 122b and the second aperture side 124b. The primary piece 108 has a maximum distance D_{maxA} between the aperture portion 116a of the lateral boundary 112a and the first securement edge 106a of the main body 104 (illustrated in FIG. 1). The primary piece 108 also has a maximum distance D_{maxB} between the aperture portion 116b of the second lateral side 112b and the second securement edge 106b of the main body 104 (shown in FIGS. 1 and 2). The maximum distance D_{maxA} is less than the minimum width W_{minA}, and the maximum distance D_{maxB} is less than the minimum width W_{minB} such that, when the main body 104 is fully assembled, no openings between the primary piece 108, the first panel 110a, and the second panel 110b are visible. The difference between the maximum distance D_{maxA} and minimum width W_{minA}, and/or the difference between the maximum distance D_{maxB} and the minimum width W_{minB}, may be set to an optimal overlap value.

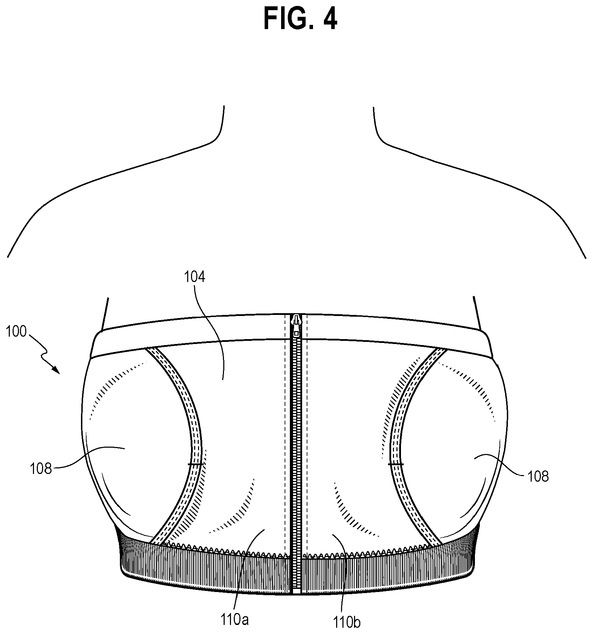

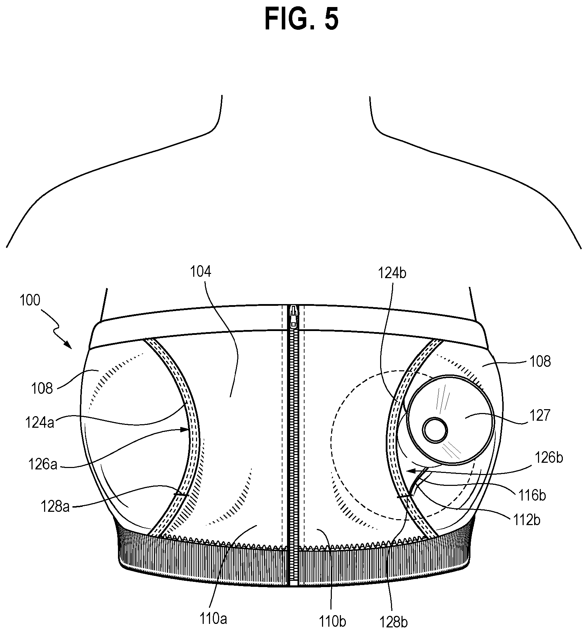

FIGS. 4 and 5 show the main body 104 once the primary piece 108, the first panel 110a, and the second panel 110 are connected. The first panel 110a overlaps the primary piece 108, and the second panel 110b also overlaps the primary piece 108. Having multiple layers (i.e., the combination of the primary piece 108 and the first panel 110a or the second panel 110b) arranged over the breasts of the wearer is beneficial because the multiple layers provide an increased elastic modulus relative to single layer areas, thereby contributing to an adaptive stretch of the pumping garment 100. The increased elastic modulus helps to support the breastmilk collection apparatus inserted into the pumping garment 100 and helps to avoid the breastmilk collection apparatus slipping downwardly away from the breast as either collection receptacle fills with breastmilk.

As illustrated in FIG. 5, the second aperture portion 116b of the second lateral boundary 112b and the second aperture side 124b of the second panel 110b together define a second aperture 126b for supporting a breast shield. That is, the second aperture portion 116b and the second aperture side 110b are free and unconnected such that insertion of a breast shield 127, preferably from an exterior of the pumping garment 100, is possible. Likewise, the first aperture portion 116a of the first lateral boundary 112a (not visible in FIG. 5 since a breast shield is not inserted) and the first aperture side 124a of the first panel 110a together define a first aperture 126a (indicated by an arrow, though not visible) for supporting a breast shield. That is, the first aperture portion 116a and the first aperture side 110a are free and unconnected such that insertion of a breast shield, preferably from an exterior of the pumping garment 100, is possible.

As illustrated in FIG. 5, elastic may be provided along the first aperture side 124a, the second aperture side 124b, the second aperture portion 116b, and/or the first aperture portion 116a (not visible in FIG. 5). The elastic may help to provide stretch to accommodate the insertion of a breast shield, such as breast shield 127 as shown in FIG. 5, further contributing to the adaptive stretch of the pumping garment 100. Alternatively, or in addition, a border material may be provided along the first aperture portion 116a, the first aperture side 124a, the second aperture portion 116b, and/or the second aperture side 124b. The border material may help the wearer identify the location of the first aperture 126a and the second aperture 126b for inserting a breast shield. Further, the border material may be a different color than the rest of the main body 104 in order to add aesthetic appeal to the pumping garment 100.

As best illustrated in FIG. 5, the second aperture portion 116b of the second lateral boundary 112b is connected to the second aperture side 124b of the second panel 110b by a bar tack 128b. Likewise, although not fully visible in FIG. 5, the first aperture portion 116a of the first lateral boundary 112a is connected to the first aperture side 124a of the first panel 110a by a bar tack 128a. The bar tacks 128a and 128b are located at the bottom of the first aperture 126a and the second aperture 126b, respectively, and help to counteract the stress this area of the pumping garment 100 experiences when breast shields are inserted into, supported by, or taken out of first aperture 126a and/or second aperture 126b.

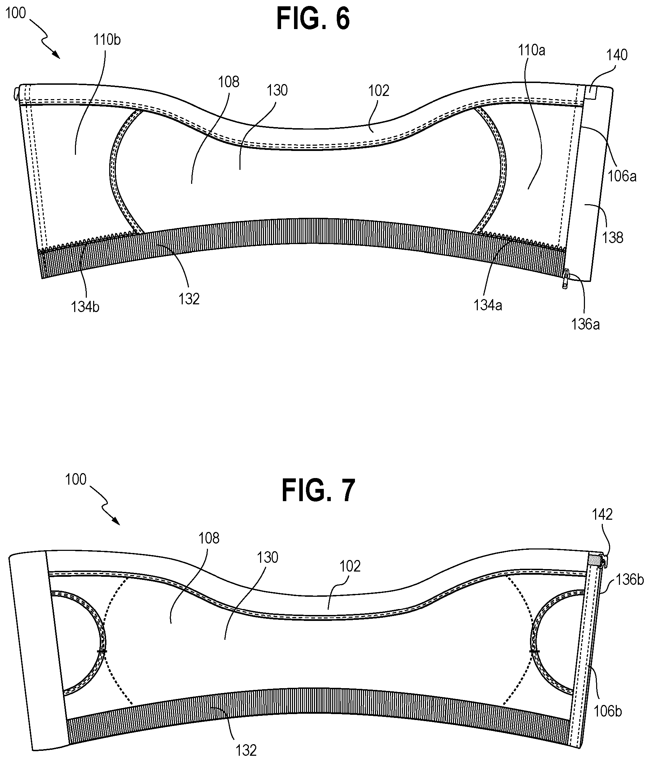

As illustrated in FIGS. 6 and 7, the primary piece 108 has an upper section 130 and a bottom band 132. The upper section 130 consists of a singly ply of material. Because much of the pumping garment 100 is only a single ply of material thick as a result of the upper section 130 using a single ply of material, the weight of the pumping garment 100 is reduced. The bottom band 132 is seamlessly connected to the upper section and comprises a 2-ply material. That is, the bottom band 132 is knit into the primary piece 108 through the manufacturing process in a manner that does not require an exposed seam. The unitary construction of the bottom band 132 with the integral upper section 130 simplifies the manufacturing process by eliminating the need to sew a separate bottom band into the bustier while enhancing the comfort of the bustier by eliminating a seam that would otherwise exist along the entire length of bottom band 132. The bottom band 132 helps to hold the pumping garment 100 against the rib cage area of the wearer. The primary piece 108 can be formed of a tubular knit material manufactured, for example, on a circular knitting machine, a flat knitting machine, or on a Warp knitting machine. The bottom band 132 may comprise a ribbed material.

The top band 102, also illustrated in FIGS. 6 and 7, is separately attached to the primary piece 108. The top band 102 can be formed of a suitable, stretchable material, such as a wide strip of elastic, further contributing to the adaptive stretch of the pumping garment 100. The top band 102 ensures that the pumping garment 100 does not slip down as the weight in one or more collection receptacles increases during a pumping session.

As further illustrated in FIG. 6 the first panel 110a includes a first bottom side 134a. The bottom side 134a is sewn adjacent to and just above the bottom band 132 of the primary piece 108. Likewise, the second panel 110b includes a second bottom side 134b and is sewn adjacent to and just above the bottom band 132 of the primary piece.

As illustrated in FIG. 6, a first zipper side 136a and a zipper facing 138 is connected to the first securement edge 106a. As illustrated in FIG. 7, a second zipper side 136b and is connected to the second securement edge 106b. As shown in FIG. 6, the zipper facing 138 is sufficiently wide to extend underneath both the first zipper side 136a and the second zipper side 136b when the garment is fastened and thus protects the skin of the wearer from becoming accidentally caught between or rubbed by the first zipper side 136a and the second zipper side 136b. The first zipper side 136a and the second zipper side 136b zip together to secure the pumping garment 100 around the body of a wearer.

As shown in FIGS. 6 and 7, a first fastener 140, such as a hook (which is the fastener shown) or a clip, is connected to the first securement edge 106a, and a second fastener 142, such as a clip (which is the fastener shown) or a hook, is connected to the second securement edge 106b. In particular, the first fastener 140 and the second fastener 142 may be connected adjacent the top band 102. The first fastener 142 is arranged to be connected to the second fastener 140 to help hold the pumping garment 100 in place while the first zipper side 136a and the second zipper side 136b are connected and to further help secure the pumping garment 100 during wear.

While the present disclosure has been described with respect to certain exemplary embodiments, it will be understood that variations may be made thereto and combinations can be formed therein and varied to form sub combinations that are still within the scope of the appended claims. Additionally, while a particularly-preferred embodiment is illustrated in the drawings of the present disclosure, it will be understood that the functional features disclosed and claimed herein can be accomplished in devices that differ from these drawings, and features of the drawings are not dictated by either function or by the combinations shown.

## Claims

1. A pumping garment (100) comprising:
a top band (102);
a main body (104) below the top band (102), the main body (104) including a primary piece, a panel (110a; 110b) overlapping the primary piece (108), and a securement edge (106a; 106b);
the pumping garment (100) further comprising:
the primary piece (108) including a lateral boundary (112a; 112b) having a securement portion (114a; 114b) extending along the securement edge (106a; 106b) of the main body (104) and an aperture portion (116a; 116b) extending away from the securement edge (106a; 106b) of the main body (104), and
the panel (110a; 110b) including a securement side (122a; 122b) extending along the securement edge (106a; 106b) of the main body (104) and an aperture side (124a; 124b), and **characterized in that** the securement portion (114a; 114b) and aperture portion (116a; 116b) define the lateral boundary (112a; 112b) of the primary piece (108); and
the securement side (122a; 122b) and aperture side (124a; 124b) define opposite edges of the panel (110a; 110b), and the aperture side (124a; 124b) is spaced away from the securement edge (106a; 106b) of the main body (104);
the aperture portion (116a; 116b) of the lateral boundary (112a; 112b) of the primary piece (108) and the aperture side (124a; 124b) of the panel (110a; 110b) together defining an aperture for supporting a breast shield (127).

2. The pumping garment (100) of claim 1,
the panel of the main body (104) including a first panel (110a) overlapping the primary piece (108) and a second panel (110b) overlapping the primary piece (108),
the securement edge of the main body (104) including a first securement edge (106a), and a second securement edge (106b);
the primary piece (108) including
a first lateral boundary (112a) having a first securement portion (114a) extending along the first securement edge (106a) of the main body (104) and a first aperture portion (116a) extending away from the first securement edge (106a) of the main body (104);
a second lateral boundary (112b) having a second securement portion (114b) extending along the second securement edge (106b) of the main body (104) and a second aperture portion (116b) extending away from the second securement edge (106b) of the main body (104);
the first panel (110a) including a first securement side (122a) extending along the first securement edge (106a) of the main body (104) and a first aperture side (124a) spaced away from the first securement edge (106a) of the main body (104); and
the second panel (110b) including a second securement side (122b) extending along the second securement edge (106b) of the main body (104) and a second aperture side (124b) spaced away from the second securement edge (106b) of the main body (104);
the first aperture portion (116a) of the first lateral boundary (112a) of the primary piece (108) and the first aperture side (124a) of the first panel (110a) together defining a first aperture for supporting a breast shield (127);
the second aperture portion (116b) of the second lateral boundary (112b) of the primary piece (108) and the second aperture side (124b) of the second panel (110b) together defining a second aperture for supporting a breast shield (127).

3. The pumping garment (100) of claim 1, and the aperture portion (116a; 116b) of the lateral boundary (112a; 112b) of the primary piece (108) curves away from the securement edge (106a; 106b) of the main body (104) to form a symmetric arc.

4. The pumping garment (100) of claim 1, and the securement portion (114a; 114b) of the lateral boundary (112a; 112b) of the primary piece (108) extends along the securement edge (106a; 106b) of the main body (104) for a first distance (D_{1A}; D_{1B}) beginning at a top (118a; 118b) of the securement edge (106a; 106b) and for a second distance (D_{2A}; D_{2B}) beginning at a bottom (120a; 120b) of the securement edge (106a; 106b).

5. The pumping garment (100) of claim 4, and the aperture portion (116a; 116b) of the lateral boundary (112a; 112b) of the primary piece (108) is located between the first distance (D_{1A}; D_{1B}) and the second distance (D_{2A}; D_{2B}).

6. The pumping garment (100) of claim 1, and the aperture side (124a; 124b) of the panel (110a; 110b) curves toward the securement side (122a; 122b) of the panel (110a; 110b).

7. The pumping garment (100) of claim 1, and a maximum distance between the aperture portion (116a; 116b) of the lateral boundary (112a; 112b) of the primary piece (108) and the securement edge (106a; 106b) of the main body (104) being less than a minimum width (W_{minA}; W_{minB}) of the panel (110a; 110b).

8. The pumping garment (100) of claim 1, and the aperture portion (116a; 116b) of the lateral boundary (112a; 112b) of the primary piece (108) is connected to the aperture side (124a; 124b) of the panel (110a; 110b) by a bar tack (128a; 128b).

9. The pumping garment (100) of claim 2, a first zipper side (136a) and a zipper facing (138) connected to the first securement edge (106a), and a second zipper side (136b) connected to the second securement edge (106b).

10. The pumping garment (100) of claim 1, the pumping garment (100) configured for adaptive stretch as a result of at least one of an elastic modulus created by the overlapping of the primary piece (108) and the panel (110a; 110b), elastic provided along the aperture side (124a; 124b), elastic provided along the aperture portion (116a; 116b), and the top band (102) being formed of elastic.

## Patentansprüche

1. Pumpkleidungsstück (100), umfassend:
ein oberes Band (102),
einen Hauptkörper (104) unter dem oberen Band (102), wobei der Hauptkörper (104) einen primären Teil, eine Stoffbahn (110; 110b), die den primären Teil (108) überlappt, und einen Verschlussrand (106a; 106b) umfasst,
wobei das Pumpkleidungsstück (100) weiterhin umfasst:
den primären Teil (108) einschließlich einer lateralen Grenze (112a; 112b) mit einem Verschlussteil (114a; 114b), der sich entlang des Verschlussrands (106a; 106b) des Hauptkörpers (104) erstreckt, und eines Öffnungsteils (116a; 116b), der sich von dem Befestigungsrand (106a; 106b) des Hauptkörpers (104) weg erstreckt
die Stoffbahn (110a; 110b) einschließlich einer Verschlussseite (122a; 122b), die sich entlang des Verschlussrands (106a; 106b) des Hauptkörpers (104) erstreckt, und einer Öffnungsseite (124a; 124b),
**dadurch gekennzeichnet, dass**:
der Verschlussteil (114a, 114b) und der Öffnungsteil (116a; 116b) die laterale Grenze (112a; 112b) des primären Teils (108) definieren,
die Verschlussseite (122a; 122b) und die Öffnungsseite (124a; 124b) gegenüberliegende Ränder der Stoffbahn (110a; 110b) definieren und die Öffnungsseite (124a; 124b) von dem Verschlussrand (106a; 106b) des Hauptkörpers (104) beabstandet ist, und
der Öffnungsteil (116a; 116b) der lateralen Grenze (112a; 112b) des primären Teils (108) und die Öffnungsseite (124a; 124b) der Stoffbahn (110a; 110b) gemeinsam eine Öffnung für das Halten einer Brusthaube (127) definieren.

2. Pumpkleidungsstück (100) nach Anspruch 1, wobei:
die Stoffbahn des Hauptkörpers (104) eine erste Stoffbahn (110a), die mit dem primären Teil (108) überlappt, und eine zweite Stoffbahn (110b), die mit dem primären Teil (108) überlappt, umfasst,
der Verschlussrand des Hauptkörpers (104) einen ersten Verschlussrand (106a) und einen zweiten Verschlussrand (106b) umfasst,
der primäre Teil (108) umfasst:
eine erste laterale Grenze (112a) mit einem ersten Verschlussteil (114a), der sich entlang des ersten Verschlussrands (106a) des Hauptkörpers (104) erstreckt, und mit einem ersten Öffnungsteil (116a), der sich von dem ersten Verschlussrand (106a) des Hauptkörpers (104) weg erstreckt,
eine zweite laterale Grenze (112b) mit einem zweiten Verschlussteil (114b), der sich entlang des zweiten Verschlussrands (106b) des Hauptkörpers (104) erstreckt, und mit einem zweiten Öffnungsteil (116b), der sich von dem zweiten Verschlussrand (106b) des Hauptkörpers (104) weg erstreckt,
die erste Stoffbahn (110a) eine erste Verschlussseite (122a), die sich entlang des ersten Verschlussrands (106a) des Hauptkörpers (104) erstreckt, und eine erste Öffnungsseite (124a), die von dem ersten Verschlussrand (106a) des Hauptkörpers (104) beabstandet ist, umfasst,
die zweite Stoffbahn (110b) eine zweite Verschlussseite (122b), die sich entlang des zweiten Verschlussrands (106b) des Hauptkörpers (104) erstreckt, und eine zweite Öffnungsseite (124b), die von dem zweiten Verschlussrand (106b) des Hauptkörpers (104) beabstandet ist, umfasst,
der erste Öffnungsteil (116a) der ersten lateralen Grenze (112a) des primären Teils (108) und die erste Öffnungsseite (124a) der ersten Stoffbahn (110a) gemeinsam eine erste Öffnung für das Halten einer Brusthaube (127) definieren, und
der zweite Öffnungsteil (116b) der zweiten lateralen Grenze (112b) des primären Teils (108) und die zweite Öffnungsseite (124b) der zweiten Stoffbahn (110b) gemeinsam eine zweite Öffnung für das Halten einer Brusthaube (127) definieren.

3. Pumpkleidungsstück (100) nach Anspruch 1, wobei sich der Öffnungsteil (116a; 116b) der lateralen Grenze (112a; 112b) des primären Teils (108) von dem Verschlussrand (106a; 106b) des Hauptkörpers (104) weg krümmt, um einen symmetrischen Bogen zu bilden.

4. Pumpkleidungsstück (100) nach Anspruch 1, wobei sich der Verschlussteil (114a; 114b) der lateralen Grenze (112a; 112b) des primären Teils (108) entlang des Verschlussrands (106a; 106b) des Hauptkörpers (104) über eine erste Distanz (D_{1A}; D_{1B}) beginnend an einer oberen Seite (118a; 118b) des Verschlussrands (106a; 106b) und über eine zweite Distanz (D_{2A}; D_{2B}) beginnend an einer unteren Seite (120a; 120b) des Verschlussrands (106a; 106b) erstreckt.

5. Pumpkleidungsstück (100) nach Anspruch 4, wobei der Öffnungsteil (116a; 116b) der lateralen Grenze (112a; 112b) des primären Teils (108) zwischen der ersten Distanz (D_{1A}; D_{1B}) und der zweiten Distanz (D_{2A}; D_{2B}) angeordnet ist.

6. Pumpkleidungsstück (100) nach Anspruch 1, wobei sich die Öffnungsseite (124a; 124b) der Stoffbahn (110a; 110b) zu der Verschlussseite (122a; 122b) der Stoffbahn (110a; 110b) krümmt.

7. Pumpkleidungsstück (100) nach Anspruch 1, wobei eine maximale Distanz zwischen dem Öffnungsteil (116a; 116b) der lateralen Grenze (112a, 112b) des primären Teils (108) und dem Verschlussrand (106a; 106b) des Hauptkörpers (104) kleiner als eine minimale Breite (W_{minA}; W_{minB}) der Stoffbahn (110a; 110b) ist.

8. Pumpkleidungsstück (100) nach Anspruch 1, wobei der Öffnungsteil (116a; 116b) der lateralen Grenze (112a; 112b) des primären Teils (108) mit der Öffnungsseite (124a; 124b) der Stoffbahn (110a; 110b) durch eine Riegelnaht (128a; 128b) verbunden ist.

9. Pumpkleidungsstück (100) nach Anspruch 2, das eine erste Reißverschlussseite (136a), und einen Reißverschlussbeleg (138), die mit dem ersten Verschlussrand (106a) verbunden sind, und eine zweite Reißverschlussseite (136b), die mit dem zweiten Verschlussrand (106b) verbunden ist, umfasst.

10. Pumpkleidungsstück (100) nach Anspruch 1, wobei das Pumpkleidungsstück (100) für eine adaptive Dehnung aufgrund eines durch das Überlappen des primären Teils (108) und der Stoffbahn (110a; 110b) erzeugten Elastikmoduls, eines entlang der Öffnungsseite (124a; 124b) vorgesehenen elastischen Materials, eines entlang des Öffnungsteils (116a; 116b) vorgesehenen elastischen Materials und/oder des aus einem elastischen Material ausgebildeten oberen Bands (102) konfiguriert ist.

## Revendications

1. Vêtement de tirage de lait (100) comprenant :
une bande supérieure (102) ;
un corps principal (104) en-dessous de la bande supérieure (102), le corps principal (104) incluant un élément primaire, un panneau (110a ; 110b) chevauchant l'élément primaire (108), et un bord de sécurisation (106a ; 106b) ;
le vêtement de tirage de lait (100) comprenant en outre :
l'élément primaire (108) incluant une limite latérale (112a ; 112b) ayant une portion de sécurisation (114a ; 114b) s'étendant le long du bord de sécurisation (106a ; 106b) du corps principal (104) et une portion d'ouverture (116a ; 116b) s'étendant à l'opposé du bord de sécurisation (106a ; 106b) du corps principal (104), et
le panneau (110a ; 110b) incluant un côté de sécurisation (122a ; 122b) s'étendant le long du bord de sécurisation (106a ; 106b) du corps principal (104) et un côté d'ouverture (124a ; 124b), et
**caractérisé en ce que** la portion de sécurisation (114a ; 114b) et la portion d'ouverture (116a ; 116b) définissent la limite latérale (112a ; 112b) de l'élément primaire (108) ; et
le côté de sécurisation (122a ; 122b) et le côté d'ouverture (124a ; 124b) définissent des bords opposés du panneau (110a ; 110b), et le côté d'ouverture (124a ; 124b) est espacé à l'opposé du bord de sécurisation (106a ; 106b) du corps principal (104) ;
la portion d'ouverture (116a ; 116b) de la limite latérale (112a ; 112b) de l'élément primaire (108) et le côté d'ouverture (124a ; 124b) du panneau (110a ; 110b) définissant ensemble une ouverture pour supporter une protection de sein (127).

2. Vêtement de tirage de lait (100) selon la revendication 1,
le panneau du corps principal (104) incluant un premier panneau (110a) chevauchant l'élément primaire (108) et un second panneau (110b) chevauchant l'élément primaire (108),
le bord de sécurisation du corps principal (104) incluant un premier bord de sécurisation (106a), et un second bord de sécurisation (106b) ;
l'élément primaire (108) incluant
une première limite latérale (112a) ayant une première portion de sécurisation (114a) s'étendant le long du premier bord de sécurisation (106a) du corps principal (104) et une première portion d'ouverture (116a) s'étendant à l'opposé du premier bord de sécurisation (106a) du corps principal (104) ;
une seconde limite latérale (112b) ayant une seconde portion de sécurisation (114b) s'étendant le long du second bord de sécurisation (106b) du corps principal (104) et une seconde portion d'ouverture (116b) s'étendant à l'opposé du second bord de sécurisation (106b) du corps principal (104) ;
le premier panneau (110a) incluant un premier côté de sécurisation (122a) s'étendant le long du premier bord de sécurisation (106a) du corps principal (104) et un premier côté d'ouverture (124a) espacé à l'opposé du premier bord de sécurisation (106a) du corps principal (104) ; et
le second panneau (110b) incluant un second côté de sécurisation (122b) s'étendant le long du second bord de sécurisation (106b) du corps principal (104) et un second côté d'ouverture (124b) espacé à l'opposé du second bord de sécurisation (106b) du corps principal (104) ;
la première portion d'ouverture (116a) de la première limite latérale (112a) de l'élément primaire (108) et le premier côté d'ouverture (124a) du premier panneau (110a) définissant conjointement une première ouverture pour supporter une protection de sein (127) ;
la seconde portion d'ouverture (116b) de la seconde limite latérale (112b) de l'élément primaire (108) et le second côté d'ouverture (124b) du second panneau (110b) définissant conjointement une seconde ouverture pour supporter une protection de sein (127).

3. Vêtement de tirage de lait (100) selon la revendication 1, et la portion d'ouverture (116a ; 116b) de la limite latérale (112a ; 112b) de l'élément primaire (108) s'incurve à l'opposé du bord de sécurisation (106a ; 106b) du corps principal (104) pour former un arc symétrique.

4. Vêtement de tirage de lait (100) selon la revendication 1, et la portion de sécurisation (114a ; 114b) de la limite latérale (112a ; 112b) de l'élément primaire (108) s'étend le long du bord de sécurisation (106a ; 106b) du corps principal (104) sur une première distance (D_{1A} ; D_{1B}) commençant au niveau d'un sommet (118a ; 118b) du bord de sécurisation (106a ; 106b) et sur une seconde distance (D_{2A} ; D_{2B}) commençant au niveau d'un fond (120a ; 120b) du bord de sécurisation (106a ; 106b).

5. Vêtement de tirage de lait (100) selon la revendication 4, et la portion d'ouverture (116a ; 116b) de la limite latérale (112a ; 112b) de l'élément primaire (108) est localisée entre la première distance (D_{1A} ; D_{1B}) et la seconde distance (D_{2A} ; D_{2B}).

6. Vêtement de tirage de lait (100) selon la revendication 1, et le côté d'ouverture (124a ; 124b) du panneau (110a ; 110b) s'incurve vers le côté de sécurisation (122a ; 122b) du panneau (110a ; 110b).

7. Vêtement de tirage de lait (100) selon la revendication 1, et une distance maximale entre la portion d'ouverture (116a ; 116b) de la limite latérale (112a ; 112b) de l'élément primaire (108) et le bord de sécurisation (106a ; 106b) du corps principal (104) étant inférieure à une largeur minimale (W_{minA} ; W_{minB}) du panneau (110a ; 110b).

8. Vêtement de tirage de lait (100) selon la revendication 1, et la portion d'ouverture (116a ; 116b) de la limite latérale (112a ; 112b) de l'élément primaire (108) est raccordée au côté d'ouverture (124a ; 124b) du panneau (110a ; 110b) par une bride d'arrêt (128a ; 128b).

9. Vêtement de tirage de lait (100) selon la revendication 2, un premier côté de fermeture à glissière (136a) et une face de fermeture à glissière (138) raccordée au premier bord de sécurisation (106a), et un second côté de fermeture à glissière (136b) raccordé au second bord de sécurisation (106b).

10. Vêtement de tirage de lait (100) selon la revendication 1, le vêtement de tirage de lait (100) configuré pour un étirement adaptatif en résultat d'au moins l'un d'un module élastique créé par le chevauchement de l'élément primaire (108) et du panneau (110a ; 110b), élastique prévu le long du côté d'ouverture (124a ; 124b), élastique prévu le long de la portion d'ouverture (116a ; 116b), et la bande supérieure (102) étant constituée d'élastique.
